# EUROPEAN PATENT APPLICATION

(11) **EP 1 967 218 A2**
(43) Date of publication of application: **10.09.2008**
(21) Application number: 08101292.4
(22) Date of filing: 05.02.2008
(51) Int. Cl.: A61L 27/06, A61L 27/50, A61L 31/02, A61L 31/14

(54) **Nanostructured titanium alloys for use as biomaterials in the manufacture of medical devices**

(30) Priority: 05.02.2007 IT MI20070190
(71) Applicant: LIMA LTO S.P.A., 33030 Villanova di San Daniele del Friuli (UD) (IT)
(72) Inventor: Ziche, Marina, 33030 VILLANOVA DI SAN DANIELE DEL FRIULI (UD) (IT); Facchini, Alessandro, 33170 PORDENONE (IT)
(74) Representative: Minoja, Fabrizio

(57) **Abstract**

Disclosed are implantable medical devices consisting of nanostructured titanium alloys.

## Description

This invention relates to implantable medical devices made of nanostructured titanium alloys.

### INTRODUCTION

Cardiovascular diseases are the main cause of mortality in the industrialised countries. Some of them, such as heart valve disorders, myocardial infarction, atherosclerosis, and ischaemia of the myocardium and lower limbs, are increasingly often treated by surgery involving the use of replacement or supporting medical devices. Some examples are heart valves and stents. Stents, which are commonly implanted after angioplasty (an operation that removes occlusions of the blood vessels caused by the formation of atheromata, and enables the blood flow to be maintained), perform two important functions. Firstly, they promote the recovery of the endothelium, a tissue lining the blood vessels, the operation of which is essential to the health of the blood vessels and bloodstream. Secondly, they prevent excessive accumulation of smooth muscle cells in the blood vessels and frequent re-occlusion of the vessels (a process known as re-stenosis).

Modifications have been introduced into metal stents in recent years by coating their surface with drugs which are vasoprotective or toxic to the smooth muscle component (known as medicated stents); however, the results have been of doubtful clinical efficacy, and sometimes very poor.

The unsolved problems of traditional or medicated stents are:
- the formation of blood clots, which can cause thrombosis, and the consequent need to administer anticoagulant drugs to patients;
- a foreign-body reaction or allergic reaction to the material with which the device is forged.

To eliminate these problems, research in this sector is focusing firstly on formulating alloys which do not contain toxic or allergenic metals like nickel and aluminium, and secondly on modifying the surfaces of devices to facilitate biocompatibility with the cells (especially endothelial stem cells or differentiated cells).

Many cardiovascular and medical implants are manufactured from metal alloys, which provide greater biocompatibility and haemocompatibility than other materials.

Metal biomaterials can be obtained from simple metals and their composition in alloys, obtained from a basic metal with the addition of metallic and non-metallic elements. Biomaterials are heat-treated in order to acquire given mechanical properties, and surface-finished to maximise their biocompatibility.

These biomaterials are characterised by their ability to bear heavy loads without breaking or deforming, and also withstand mechanical fatigue very well. They are consequently used in the manufacture of orthopaedic devices. However, when they come into contact with biological fluids, they are liable to wear.

The first metal used as a biomaterial was vanadium, used to make fixing plates for bone fractures. Stainless steel, which is stronger and less liable to corrosion than its predecessor, was introduced later; it is an alloy consisting of iron, carbon and a smaller percentage of chromium. The main type of stainless steel used is austenitic, which is more suitable for clinical use in prostheses due to its high corrosion-resistance (stainless steel 316L, Cr-Ni-Mo steel with a very low C content).

There are various types of cobalt alloys suitable to be implanted, which differ in terms of the amount of the various elements they contain; the two most frequently used are CoCrMo (cobalt-chromium-molybdenum) alloys and CoNiCrMo (cobalt-nickel-chromium-molybdenum) alloys. The latter possesses considerably greater mechanical strength than the former, and is therefore suitable for applications in which high resistance to mechanical fatigue and lengthy use is required, such as artificial hip joints where, in additional to the functional stress on this important joint, long-term wear of the prosthesis needs to be prevented.

Titanium, the latest metal to be used for implants, has proved advantageous due to its lightness and good mechanical properties. The type most commonly used is the alloy Ti6Al4V (titanium-aluminium-vanadium), which consists of 6.00% aluminium and 4.00% vanadium. It possesses good corrosion resistance due to the formation of an oxidised surface layer; however, it has low resistance to corrosion caused by rubbing, and is therefore not used for joint surfaces. An important characteristic of titanium and its alloy Ti6Al4V (Ahmad et al.,1999) is its ability to stimulate the production of protein substances, mineralised substances and components of extracellular matrix, which makes this alloy very important for bone regeneration (Rack and Qazi, 2006). Nitinol, an alloy of titanium and nickel, possesses the important characteristic of shape-retention memory or elastic memory, namely the ability to resume its original shape after plastic deformation, with an increase in temperature. These particular characteristics make this alloy particularly suitable for the production of vena cava filters, cardiovascular stents and orthopaedic implants.

Endothelial cells have a low replication rate under physiological conditions. This biological characteristic for the aging the cell population and for the loss of the vascular functions underlying various diseases. In chronic disorders and conditions of traumatic destruction of organs and tissue, the small blood vessels of the microcirculation, of which endothelium is the sole and/or prevalent cell component, are regenerated with difficulty, leading to loss of function in the organ they supply. In the event of an organ transplant and/or prosthesis, the efficacy of the result is guaranteed by appropriate revascularisation and endothelialisation.

The situation in vivo has been reproduced in the laboratory in studies conducted in vitro on endothelial cells. These studies demonstrate that the growth and survival of the endothelium can be modified by the composition of the culture surfaces and the availability of pro-proliferative cytokines such as fibroblastic growth factor type 2 (FGF2), a known proangiogenic factor.

### Description of the invention

It has now been discovered that the use of nanostructured titanium alloys, especially alloys containing various percentages of titanium, niobium and zirconium obtainable by forging and heat treatment from metal powders, promotes the adhesion, growth and production of high levels of growth factor FGF2 in the endothelial cells to a significantly greater extent (2 or 3 times more) than the previously known alloys.

The subject of the invention is therefore implantable medical devices consisting of nanostructured titanium alloys. Examples of said devices include cardiovascular devices, especially stents and heart valves, vena cava filters, orthopaedic devices (fixators, prostheses and the like), dental devices and, broadly speaking, devices implantable in situations in which it is desirable to promote endothelial cell proliferation and angiogenesis. Another example of application of the invention is the use of said devices to promote the endothelial production of proangiogenic growth factors, such as FGF2, under pathological conditions and conditions in which revascularisation is deficient (such as diabetes, circulatory failure and genetic disorders).

The efficiency of the alloys according to the invention has been evaluated with biological tests which measure the proliferation rate of the endothelial cells and their functions, such as production and organisation of cytoskeletal proteins, and production of growth factors and the molecules responsible for vasodilating action.

The production method does not impose any limits on the chemical compositions, so elements in the alloy which are potentially harmful to the human body can be replaced by alternative elements which guarantee total biocompatibility. The results were obtained by a combination of nanostructured titanium alloy technology and forging and heat treatment processes, which also optimise their mechanical characteristics.

The nanostructured alloys based on titanium (Ti) were made and characterised by the Applicant.

The preferred biocompatible Ti alloys are those based on TiNbZr (especially compositions Ti13Nb13Zr and Ti13Nb11Zr), produced with a structure of nanometric size, ie. a crystal size smaller than 100 nm. These alloys were compared with the alloy currently on the market, Ti6Al4V, produced both in the commercial structure (standard) and the nanometric structure. This structure was obtained by an innovative manufacturing process wherein alloys consisting of crystals of nanometric dimensions (approx. 20 nm) are produced by mechanical fabrication.

The high-energy mechanical fabrication process used to produce metal powders with a nanometric structure is performed, for example, with the equipment described in EP 665770.

X-ray analysis of alloy Ti13Nb13Zr demonstrates that the material produced consists of Ti in both its phases α and β. Analysis of the difference in density between the powders and the material demonstrates that the alloy produced has a good degree of compacting. When the data relating to mechanical tests on nanostructured Ti13Nb13Zr were compared, the hardness values proved to be greater than with either nanostructured or standard alloy Ti6Al4V.

Forging tests were conducted to establish the hot workability characteristics of the alloy and optimise the parameters required for the moulding process.

The heating temperatures most suitable for both test materials were identified (940°C for nanostructured Ti6Al4V and 850°C for nanostructured Ti13Nb11Zr); Ti13Nb11Zr demonstrated particular characteristics of flowability and deformability.

Microstructural tests performed on forged materials demonstrate that the grain distribution of both alloys is more compact and homogenous.

The mechanical properties of the new nanostructured materials, when subjected to a forging process, are better for both materials than with untreated materials; in particular, as regards Ti13Nb11Zr, the material showed a considerable improvement in that it was less fragile.

Tests were conducted on the effect of heat treatments after the forging stage, such as tempering, stress relieving and STA (Solution Treatment and Aging) at different temperatures.

The mechanical properties of the materials were further improved in this way.

The materials were hot-forged in the form of bars, from which discs or squares were obtained and then washed, packaged and sterilised with β rays for the biological tests.

All the studies reported used endothelial cells of the microcirculation (the main site of vascular remodelling and the angiogenesis process) isolated from bovine coronary venular endothelial cells (CVEC) (Schelling et a1.,1988). CVECs are cultured in plates with a 10 cm diameter, coated with 1% gel (w/v in water) and maintained in the culture with Dulbecco's Modified Eagles Medium (DMEM, Sigma-Aldrich, Milan), containing 1000 mg/ml of D-glucose. 100 µg/ml of penicillin, 100 µg/ml of streptomycin and 2 mM of L-glutamine is added to the medium. Bovine Calf Serum (BCS) (Hyclone, Logan, UT), at a concentration of 10% of the total volume (complete medium), is added to the culture medium to mimic and allow the best growth conditions. The cells are maintained in a humidified incubator at the temperature of 37°C, in the presence of 5% CO₂, until confluence. The CVECs are then propagated, by detachment with trypsin/EDTA, and 1:3 dilution every 48 hours.

Under confluence conditions CVECs present contact inhibition, and have a polygonal morphology and highly flattened cells. A spindle-shaped or punctiform morphology indicates cell damage.

They are identified by the presence of antigen factor VIII, incorporation of LDL-acetylate and expression of membrane integrin receptor αvβ3.

The salient biochemical characteristics of this cell line, which confirm its nature as an endothelial cell, are the production of FGF-2 (Hawker and Granger, 1992), and the production of nitric oxide (Wu and Meininger, 1993) and prostacyclin in response to inflammation mediators such as bradykinin (Bachetti and Morbidelli, 2000).

### Cell survival test

The MTT viability test was used to establish the ability of the cells to survive/proliferate on biomaterials (Tonello et al., 2003). This test is an indirect method of evaluating cell growth and proliferation, because it is a colorimetric test based on the ability of metabolically active cells to convert the soluble compound 3-(4,5-dimethylthiazol-2-yl)-2,5-diphenyltetrazolium bromide (MTT, Sigma-Aldrich), via mitochondrial succinic dehydrogenase, to an insoluble formazan salt which can be determined spectrophotometrically. The optical density, measured at the wavelength of 540 nm, is proportional to the number of viable cells in the sample, and is consequently an indicator of the cytotoxicity of the material. For this test, the CVECs were seeded on the biomaterials at the concentration of 10,000 cells/well (in a volume of 500 µl) in 48-well plates. Viability was evaluated 1, 4 and 7 days after the seeding of the cells, and the medium was changed half-way through incubation for the samples due to be tested after 7 days. 4 hours before the end of the experiment the culture medium was replaced with a medium devoid of phenol red, which would otherwise have interfered with the spectrophotometric measurement, and a 5 mg/ml solution of MTT, obtained by solubilising the salt in a medium devoid of phenol red in such a way as to give a final concentration of 1.2 mM, was added. The cells were incubated at 37°C, and metabolised MTT into the insoluble derivative during this period. After 4 hours the medium was removed, the formazan salt was solubilised with DMSO (dimethyl sulphoxide), and its concentration was determined by spectrophotometric reading at 540 nm (Spectrafluor, Tecan). Results obtained at different incubation times are shown as absorbance (Abs) at 540 nm.

### Analysis of morphology and cell adhesion

Endothelial cells are particularly sensitive to stimuli from the surrounding environment, and alterations in it modify the polygonal morphology and adhesion capacity of the cells, with alterations in their angiogenetic properties. Under these circumstances, the endothelial cell adhesion and morphology study represents an indicator of the biocompatibility of the material under test. Although microscopy techniques are generally used for that purpose, we preferred to use the classic Scanning Electron Microscope (SEM) or the Environmental Scanning Electron Microscope (ESEM), because they also demonstrate the nanometric structures of the materials.

### Scanning Electron Microscopy (SEM) and Environmental Scanning Electron Microscopy (ESEM)

To evaluate the degree of adhesion and morphology with SEM (Philips XL20, Netherlands), endothelial cells were seeded at the concentration of 7,500 cells on each test material, which was placed in a 24-well plate and incubated at 37°C. The control is represented by cells seeded on 1 cm slide covers. After incubation the cells are washed three times, for 5 minutes per wash, with phosphate buffer (PBS, Phosphate Buffered Saline), Ca²⁺ and Mg²⁺ to remove the medium completely, and then fixed with 2.5% glutaraldehyde in 100 nM sodium cacodylate for 1 hour at 4°C (Pezzatini et al., 2006). The samples are then washed for 18 hours with 100 nM sodium cacodylate and post-fixed with 1% OsO₄ in 200 nM Na cacodylate at 4°C for 2 hours. After fixing, the samples are dehydrated with serial dilutions of ethanol, and then dried by sublimation under vacuum with terz-butanol. After coating with 100% gold using a sputter-coater, the cells are observed under the SEM and the images acquired.

The same growth cell protocol was followed for the ESEM analysis, but the processing of the samples after incubation follows different steps. The cells are washed three times with PBS, then fixed with 2.5% glutaraldehyde in PBS buffer for 1 hour at ambient temperature. After fixing, the samples are washed three times with PBS, for 5 minutes each wash, and immediately observed under the electron microscope.

### Immunofluorescence

Immunofluorescence tests were conducted to evaluate the expression and localisation of proteins which are important to the morphology, adhesion and proliferation of the endothelium, and consequently involved in the angiogenetic response of the cells to the materials under study. In particular, the expression and organisation of cytoskeletal proteins (β-actin) and adhesion proteins (integrin αvβ3) were studied. The expression of biochemical parameters important for the endothelial and angiogenetic functions, such as fibroblast growth factor (FGF-2), endothelial cell nitric oxide synthase (ecNOS) and cyclooxygenase-2 (COX-2) was also evaluated.

The CVECs were seeded in a medium containing 10% serum at a density of 7,500 cells/well in 24-well plates, on the base of which the discs of the alloys under study were placed. The cells were left to grow for 4 days at 37°C (Pezzatini et al, 2006).

After incubation, the cells were washed 3 times, for 5 minutes each wash, with PBS containing Ca²⁺ and Mg²⁺, and fixed in acetone at -20°C for 5 minutes. The slides were then washed 3 times with PBS and incubated for 45 minutes with 3% PBS-BSA at ambient temperature, to block the aspecific bonds. The cells were then incubated for 18 hours at 4°C with the primary antibody against the protein to be examined, suitably diluted in 0.5% PBS-BSA; PBS with the addition of 0.05% TWEEN 20 to permeabilise the cytoplasmic membrane was used to label the intracellular proteins (Table 1). After incubation with the primary antibody, the slides were washed 3 times for 5 minutes with 0.5% PBS-BSA, and then incubated for an hour at ambient temperature with the secondary antibody conjugated with fluorophore. The secondary antibodies used are "anti-mouse" conjugated with fluorescein (FITC) (1:50, Sigma-Aldrich) or conjugated with rhodamine (TRITC) (1:50, Sigma-Aldrich), or "anti-rabbit" conjugated with rhodamine (1:50, Sigma-Aldrich), diluted in 0.5% PBS-BSA. At the end of the incubation period, the secondary antibody was removed by 3 rinses with 0.5% PBS-BSA. The negative controls were obtained by omitting the treatment of the samples with the primary antibody.

**Table 1: Chart of proteins and the corresponding antibodies analysed by immunofluorescence. * The cytoplasmic membrane permeabilisation protocol is specified for these markers.**

| | | | |
|---|---|---|---|
| Protein | Antibody | Dilution | Manufacturer |
| Cytoskeleton | monoclonal anti-βactin | 1:40 | Sigma-Aldrich |
| Adhesion | monoclonal anti-integrin αvβ3 | 1:40 | Chemicon International, CA, USA |
| Biochemical and angiogenesis parameters | monoclonal anti-FGF-2 | 1:50* | Upstate Biotechnology, NY, USA |
| | monoclonal anti-ecNOS | 1:30* | BD Biosciences, Milan, Italy |
| | monoclonal anti-COX-2 | 1:50* | Cayman Chemical, MI, USA |

The slides were mounted with Mowioll 4-88 (Calbiochem, La Jolla, CA, USA) on slides, and the cells were observed under a Nikon Eclipse TE300 fluorescence microscope with a magnification of 40x. Various fields, chosen at random, were photographed for each sample.

The intensity of the fluorescence of the images acquired was quantified with ImageJ software 1.33u (Pezzatini et al, 2006). Three images were chosen from those acquired, and automatically converted to grey scale. 10 random cells were highlighted in each one, and the area, average intensity and minimum and maximum value of grey was measured for each one. The data were reported as mean ± SEM of the average intensity of grey recorded for the cells selected for each sample examined.

### Statistical analysis

Each experimental point is evaluated in triplicate for each experiment, and reported as mean ± SEM (standard error of the mean) of the values obtained in all the experiments performed. The data were analysed with Student's t test. Values with p<0.05 were considered statistically significant.

### RESULTS

### Nanomechanical fabrication of titanium alloys promotes endothelialisation

The biocompatibility of the nanostructured alloys Ti13Nb13Zr and Ti6Al4V for the endothelial cells of the microcirculation (CVEC) was evaluated by comparison with commercial alloy Ti6Al4V under standard cell growth conditions on polystyrene for cell cultures or glass (for the immunofluorescence and SEM tests).

The alloys were characterised by their potential cytotoxicity and their ability to sustain/induce endothelial proliferation, cell viability being evaluated by the MTT colorimetric test, according to the growth time. The alloys proved not to be cytotoxic. Moreover, the nanostructured surfaces promote endothelial growth and significantly increase (p<0.05) cell survival and proliferation after 4 and 7 days' growth, when the entire surface of the nanostructured alloy discs is covered with endothelial cells.

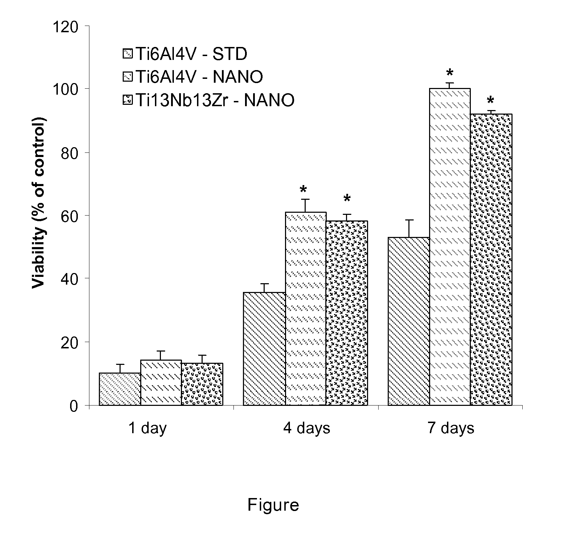

The Figure shows the % viability of endothelial cells grown on the alloys compared with the control, in which the cells were grown on polystyrene. The endothelial cells are incubated for 1, 4 and 7 days with the titanium alloys in medium containing 10% serum, and the viability is measured using the MTT test. The data are reported as mean ± SEM of n=5 experiments conducted in duplicate.
* p<0.05 vs Ti6Al4V.

As shown in Table 2, after 7 days in the culture the number of endothelial cells present on the nanostructured discs increases to 7 times more than the control, and twice as many as the standard alloys.

**Table 2: Endothelial proliferation on metal alloy discs after 7 days' culture. The total number of cells was calculated on the basis of metabolic activity measured with the MTT assay and reprocessed with a calibration curve (absorbance at 540 nm/number of cells). N=5 experiments conducted in duplicate.**

| | Number of cells/sample | p vs control |
|---|---|---|
| Control | 2x10⁴ | |
| standard Ti6Al4V | 7x10⁴ | <0,05* |
| nano Ti6Al4V | 1,56x10⁵ | <0,01** |
| nano Ti13Nb13Zr | 1,53x10⁵ | <0,01** |

The cell morphology, evaluated with SEM microscopy of CVECs after 4 days' incubation on the alloys, demonstrates the acquisition of a normal polygonal endothelial morphology with good adhesion to the substrate, connections between adjacent cells, and achievement and maintenance of confluence. The number of cells observed on the alloys was greater than on the control, confirming the findings made with the MTT test.

The expression and localisation of membrane integrin αvβ3 involved in endothelial adhesion and survival, and organisation of the structural proteins (β-actin) of the cytoskeleton, was evaluated by immunofluorescence reactions after 4 days' growth of the cells on the samples.

Titanium alloys do not alter the adhesion capacity of the endothelial cells, and the expression and localisation of integrin αvβ3 is maintained. In fact, the organisation of integrin is observed in the focal contacts of the cytoplasmic membrane in both the control and the alloys.

In cultured cells, β-actin is localised on the edge of the cytoplasm, forming a dense periplasmic ring. When cells are grown on nanostructured alloys, the actin reorganises to form stress fibres with a transcytoplasmic arrangement, demonstrating mitogenic activation of the endothelium by the biomaterial.

These results indicate that titanium alloys promote cell adhesion and proliferation, and that nanostructured alloys have a greater ability to induce the activation of a phenotype with high replication capacity in the endothelium.

In order to use these materials to make prostheses it is necessary to establish whether they affect the vascular functions or induce an inflammatory or thrombogenic process. Consequently, the endothelium was evaluated in terms of its ability to maintain the vasodilating and vasoprotective function (such as ecNOS) and not to acquire pro-inflammatory and thrombogenic capacity (like cyclooxygenase-2, COX-2) when grown on nanostructured alloys. The two differentiating markers were evaluated by immunofluorescence after 4 days' growth, and it was observed that in the endothelial cells grown on the nanostructured alloys there was a significant increase in ecNOS expression in the perinuclear area, whereas no inducement of COX-2 compared with the control was observed.

One of the characteristics of proliferating endothelium is that it induces an angiogenetic phenotype, characterised by overexpression of cytokines and growth factors like FGF-2. When endothelial cells are grown for 4 days on nanostructured alloys, a highly significant increase in FGF-2 expression is observed (Table 3).

**Table 3. Intensity of fluorescence of protein FGF-2 in CVEC grown on**

| | FGF-2/cell (Intensity of fluorescence) | SEM | p |
|---|---|---|---|
| Control | 3,6x10⁵ | 9,8x10³ | |
| standard Ti6Al4V | 7,4x10⁵ | 7,3x10³ | <0,01§ |
| nano Ti6Al4V | 9,3x10⁵ | 9,9x10³ | <0,01# |
| nano Ti13Nb13Zr | 9,6x10⁵ | 8x10³ | <0,01# |
| | | | |

the material for 4 days. The intensity of protein fluorescence was quantified on a single cell using the ImageJ programme on a sample of 30 cells (mean ± SEM). § vs control condition; # vs standard Ti6Al4V.

In particular, from the images obtained by immunofluorescence, it can be observed that the fluorescence of FGF-2 is cytoplasmic, mainly located in the perinuclear zone, and significantly increases when the cells are grown on alloys, especially nanostructured alloys. In fact, FGF2 expression measured on a single cell by fluorescence is twice as high as the control on standard alloys, and triples if the alloys are nanostructured.

In conclusion, the data demonstrate that nanostructured alloys with a standard composition (Ti6Al4V) or an innovative composition (Ti13Nb13Zr) significantly promote the functions of the vascular endothelium. Endothelial cells adhere to and proliferate on these substrates, increasing the expression and production of proangiogenic growth factors and the markers typical of endothelialisation and endothelial functionality.

### REFERENCES

Ahmad M, McCarthy MB, Gronowicz G. Biomaterials. 1999;20(3):211-20.
Bachetti T, Morbidelli L. Pharmacol Res. 2000;42(1):9-19.
Carmeliet P.. Nat Med. 2003;9(6):653-60.
Carmeliet P. Nature. 2005;438(7070):932-6.
Eliceiri BP, Cheresh DA. Cancer J Sci Am. 2000;3:S245-S249.
Hawker JR Jr, Granger HJ.. Am J Physiol. 1992;262(5 Pt 2):H1525-37.
Langer R. Biomaterials and Biomedical Engineering. Chem. Eng. Sci. 1995;50(24):4109-4121.
Parenti A, Morbidelli L, Cui XL, Douglas JG, Hood JD, Granger HJ, Ledda F, Ziche M. J Biol Chem. 1998;273:4220-6.
Pezzatini S, Solito R, Morbidelli L, Lamponi S, Boanini E, Bigi A, Ziche M. J Biomed Mater Res A 2006;76(3):656-63.
Rack HJ, Qazi JI., Materials Science & Engineering C. 2006;26(8):1269-1277.
Rousseau S, Houle F, Kotanides H, Witte L, Waltenberger J, Landry J, Huot J. J Biol Chem. 2000;275(14):10661-72.
Schelling ME, Meininger CJ, Hawker JR Jr, Granger HJ. Am J Physiol. 1988; 254(6 Pt 2):H1211-7.
Tonello C, Zavan B, Cortivo R, Brun P, Panfilo S, Abatangelo G. Biomaterials 2003; 24(7):1205-11.
Wu G, Meininger CJ. Am J Physiol. 1993 Dec;265(6 Pt 2):H1965-71.

## Claims

1. Implantable medical devices consisting of nanostructured titanium alloys.

2. Devices as claimed in claim 1, chosen from among cardiovascular devices, in particular stents and heart valves, vena cava filters, orthopaedic devices, dental devices and implantable devices in situations in which it is desirable to promote endothelial cell proliferation and vacsularization, and to the production of proangiogenic growth factors such as FGF2.

3. Devices as claimed in claim 1 or 2, wherein the titanium alloys are alloys of TiNbZr.

4. Devices as claimed in claim 3, wherein the alloys are chosen from among Ti13Nb11Zr and Ti13Nb13Zr.

5. Devices as claimed in any of claims 1 to 4, wherein the crystalline dimension of the titanium alloy is under 100 nm, and preferably approx. 20 nm.

6. Devices as claimed in any of claims 1 to 5, wherein the titanium alloys are obtained by forging and heat-treatment.

7. Use of nanostructured titanium alloys to prepare medical devices able to promote the functions of the vascular endothelium.
